(19) Europäisches Patentamt
European Patent Office
Office européen des brevets

(11) Veröffentlichungsnummer: **0 101 934**
**B1**

(12) **EUROPÄISCHE PATENTSCHRIFT**

(45) Veröffentlichungstag der Patentschrift: **23.03.88**

(21) Anmeldenummer: **83107366.3**

(22) Anmeldetag: **27.07.83**

(51) Int. Cl.[4]: **C 07 D 471/04** // A61K31/435 ,(C07D471/04, 221:00, 209:00)

(54) **Verfahren zur Herstellung von 1-(Halogenalkyl)-1,2,3,4-tetrahydro-beta-carbolinen.**

(30) Priorität: **05.08.82 DE 3229215**

(43) Veröffentlichungstag der Anmeldung:
**07.03.84 Patentblatt 84/10**

(45) Bekanntmachung des Hinweises auf die Patenterteilung:
**23.03.88 Patentblatt 88/12**

(84) Benannte Vertragsstaaten:
**AT BE CH DE FR GB IT LI NL SE**

(56) Entgegenhaltungen:
**EP-A-0 021 857**
**EP-A-0 028 381**
**EP-A-0 101 574**

**Organic Reactions 6, 152-153 (1951)**
**Advances in Heterocyclic Chemistry 3, 83-85 (1964)**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Kropp, Rudolf**
**Sprottauer Strasse 2**
**D-6703 Limburgerhof (DE)**

(56) Entgegenhaltungen:

**Patents Abstracts of Japan 6, Nr. 98 (C-106) (976), 8.6.1982**

**Chemical Communications (1968), 410**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 101 934 B1**

**Beschreibung**

Die Erfindung betrifft ein neues Verfahren zur Herstellung von 1-(Halogenalkyl)-1,2,3,4-tetrahydro-β-carbolinen.

Es ist bereits bekannt, Tryptamin mit Acetaldehyd zu 1-Methyl-1,2,3,4-tetrahydro-β-carbolin umzusetzen. Diese Umsetzung gelingt jedoch nicht mit chlorierten Acetaldehyden, wie Chloral oder Chloracetaldehyd (vgl. Advances in Heterocyclic Chemistry *3*, 83 bis 85 (1964) und Organic Reactions *6*, 151 bis 187 (1951)).

Gegenstand der Erfindung ist ein Verfahren zur Herstellung von Salzen von 1,2,3,4-Tetrahydro-β-carbolinen der Formel I

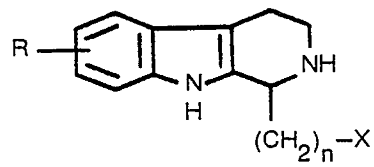

(I),

worin

R ein Wasserstoff- oder Halogenatom oder eine $C_1$—$C_4$-Alkyl-, $C_1$—$C_4$-Alkoxy- oder Trifluormethylgruppe,

X ein Halogenatom und

n eine ganze Zahl von 2 bis 5

bedeuten, dadurch gekennzeichnet, daß man ein Tryptamin der Formel II

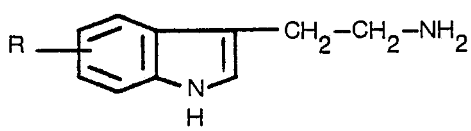

(II)

worin R die angegebene Bedeutung besitzt, oder ein Salz davon mit einem Halogenaldehyd der Formel III

$$X—(CH_2)_n—CHO \qquad \text{(III)},$$

oder einem Acetal dieser Verbindung, worin X und n die angegebene Bedeutung besitzen, nötigenfalls unter Zusatz einer Säure umsetzt.

Vorzugsweise sind R Wasserstoff, Chlor, Brom oder Trifluormethyl, X Chlor oder Brom und n 3 oder 4. Als Salze kommen vorzugsweise solche von anorganischen Säuren, wie Halogenwasserstoffsäuren, in Betracht, als Säuren die oben genannten.

Die Ausgangsstoffe III können leicht — wie in Houben-Weyl, Band 7/1, Seite 285 bis 290 beschrieben — durch katalytische Hydrierung der entsprechenden Säurechloride hergestellt werden.

Die Ausgangsstoffe II sind z.B. durch eine Fischer'sche Indolsynthese aus gegebenenfalls substituierten Arylhydrazinen und γ-amino-, γ-Halogen- oder γ-Tosyloxyaldehyden, bzw. deren Acetalen zugänglich [Sundberg: The Chemistry of Indoles, Seite 228 (1970); The Chemistry of Heterocyclic Compounds: Indoles, Part II, Seite 22 (1972)].

Bevorzugte Ausgangsstoffe II und III sind solche, in deren Formeln R Wasserstoff, Halogen, vorzugsweise Chlor und Brom, einen Alkyl- oder Alkoxyrest mit 1 bis 4 Kohlenstoffatomen oder eine Trifluormethylgruppe bedeuten, und X für Halogen, insbesondere Chlor und Brom steht. Als Acetale der Verbindungen III kommen Dialkylacetale (vorzugsweise mit bis zu 4 Kohlenstoffatomen in den Alkylresten) oder cyclische Acetale (vorzugsweise mit 5 Atomen im Ring) in Betracht.

Folgende Tryptamine können vorteilhaft als Ausgangsstoffe II verwendet werden:

3-(β-Aminoethyl)-indol, 3-(β-Aminoethyl)-5-methylindol, 3-(β-Aminoethyl)-5-chlorindol, 3-(β-Aminoethyl)-5-methoxyindol, 3-(β-Aminoethyl)-6-trifluormethylindol, 3-(β-Aminoethyl)-7-methylindol, 3-(β-Aminoethyl)-7-chlorindol sowie deren Salze, beispielsweise die Hydrochloride. Als Ausgangsstoffe III können verwendet werden β-Chlorpropionaldehyd, γ-Chlorbutyraldehyd, δ-Chlorvaleraldehyd, die entsprechenden Bromverbindungen sowie die Dimethyl-, Diethyl- und Glykolacetale.

Man setzt die Ausgangsstoffe und gegebenenfalls die Säure in ungefähr stöchiometrischer Menge miteinander um. Die Reaktion wird im allgemeinen bei einer Temperatur von 50 bis 150°C, insbesondere 70 bis 120°C, drucklos oder unter Druck, kontinuierlich oder diskontinuierlich durchgeführt. Zweckmäßig verwendet man unter den Reaktionsbedingungen inerte Lösungsmittel. Als Lösungsmittel kommen z.B. in Frage: Wasser; Alkohole wie Methanol, Ethanol, Isopropanol; Kohlenwasserstoffe wie Heptan, Toluol, Xylol; Ether wie Glykol-mono-ethylether, Tetrahydrofuran, Dioxan sowie Acetonitril, Dimethylformamid sowie Gemische der genannten Lösungsmittel. Die Säure kann dem Reaktionsgemisch zugegeben werden. Man kann aber auch die Verbindung II in Form ihres Salzes in die Reaktion einsetzen.

Die Reaktion kann wie folgt durchgeführt werden: Ein Gemisch der Ausgangsstoffe und gegebenenfalls des Lösungsmittels und Säure wird 1 bis 10 Stunden lang bei der Reaktionstemperatur

durchmischt. Beim Arbeiten in nicht-wäßrigen Systemen fällt während des Erhitzens das Reaktionsprodukt in Form seines Salzes als schwerlösliches Produkt an, das nach dem Abkühlen des Reaktionsgemisches abgesaugt oder abgeschleudert wird.

Der Ablauf der Reaktion ist überraschend, da nicht zu erwarten war, daß die Herstellung der Carboline mit halogenhaltigen Aldehyden gelingen würde.

Besonders vorteilhaft lassen sich die Verbindungen herstellen, in denen n = 3 ist. In diesem Fall kann man das Tryptamin durch das entsprechende Phenylhydrazin ersetzen, welches mit 2 Mol Halogenbutyraldehyd zum Carbolin umgesetzt wird:

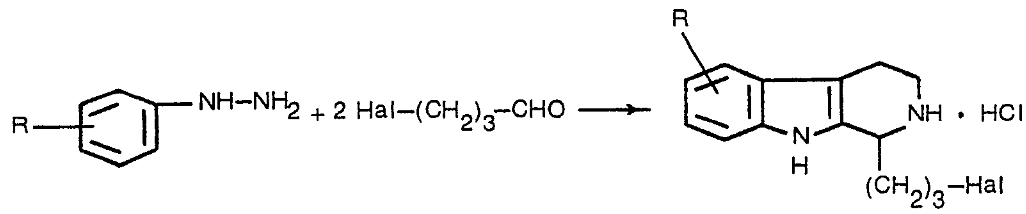

(V) (III) (I; n=3)

Man setzt die Ausgangsstoffe in ungefähr stöchiometrischer Menge oder im Überschuß zueinander um, im allgemeinen in einge Menge von 1,5 bis 10, vorteilhaft 2 bis 3 Mol Ausgangsstoff III je Mol Ausgangsstoff V.

Es können z.B. folgende Hydrazine verwendet werden: Phenyl-, o-Methylphenyl-, p-Isopropylphenyl-, p-Methylphenyl, p-Ethylphenyl, m-Trifluormethylphenyl, p-Chlorphenyl und o-Bromphenyl-hydrazin.

Als Ausgangsstoffe III können vorzugsweise γ-Chlorbutyraldehyd und γ-Brom-butyraldehyd verwendet werden.

Die Reaktion wird im allgemeinen bei einer Temperatur von 20 bis 200°C, vorzugsweise von 60 bis 120°C, drucklos oder unter Druck, kontinuierlich oder diskontinuierlich durchgeführt. Zweckmäßig verwendet man unter den Reaktionsbedingungen inerte Lösungsmittel. Als Lösungsmittel kommen z.B. in Frage: Wasser; Alkohole wie Methanol, Ethanol, Isopropanol; Kohlenwasserstoffe wie Heptan, Toluol, Xylol; Ether wie Tetrahydrofuran, Dioxan; Acetonitril, Dimethylformamid; und entsprechende Gemische.

Die Reaktion kann wie folgt durchgeführt werden: Ein Gemisch des Ausgangsstoffs und des Lösungsmittels wird während 3 bis 10 Stunden bei der Reaktionstemperatur gehalten. Je nach dem verwendeten Lösungsmittel kristallisiert das Reaktionsprodukt während des Erhitzens, während des anschließenden Abkühlens oder nach dem Abdampfen des Lösungsmittels aus und kann durch Abnutschen oder Abschleudern isoliert werden. Das Reaktionsprodukt stellt ein Salz des 1-(Halogenpropyl)-1,2,3,4-tetrahydro-β-carbolins dar.

Die neuen Verbindungen stellen wertvolle Zwischenprodukte zur Darstellung von 2-substituierten 1-(3′-Aminopropyl-1,2,3,4-tetrahydro-β-carbolinen dar.

Die folgenden Beispiele erläutern die Erfindung. Teile bedeuten Gewichtsteile.

Beispiel 1

1-(γ-Chlorpropyl)-1,2,3,4-tetrahydro-β-carbolin

a) 1280 Teile einer 20,7 gew.%igen γ-Chlorbutyraldehyd-Lösung in Xylol — hergestellt analog der in Houben-Weyl, Band 7/1, Seite 285 bis 290 beschriebenen Methode durch katalytische Hydrierung einer γ-Chlorbuttersäurechlorid-Lösung in Xylol — 490 Teile 3-(β-Aminoethyl)-indolhydrochlorid und 1000 Teile Isopropanol wurden 4 h bei 88°C gerührt. Nach dem Abkühlen wurden durch Absaugen 618 Teile (87%) 1-(γ-Chlorpropyl)-1,2,3,4-tetrahydro-β-carbolin-hydrochlorid erhalten. Nach dem Umkristallisieren aus Methanol hatte die Substanz einen Schmelzpunkt von 219 bis 221°C.

b) 950 Teile einer 11,2 gew.%igen γ-Chlorbutyraldehyd-Lösung in Xylol wurden mit 1800 Teilen Isopropanol, 200 Teilen Wasser und 54 Teilen Phenylhydrazin 6 h bei 80°C gerührt. Anschließend wurden im Wasserstrahlvakuum ca. 2000 Teile Lösungsmittel abdestilliert, der abgekühlte Rückstand abgesaugt und mit 200 Teilen Ethanol gewaschen. Man erhielt 62 Teile (43,5%) 1-(γ-Chlorpropyl)-1,2,3,4-tetrahydro-β-carbolin-hydrochlorid, Schmelzpunkt 216 bis 218°C. Nach dem Umkristallisieren aus Methanol lag der Schmelzpunkt bei 219 bis 221°C.

Analog Beispiel 1b wurden erhalten:

1-(γ-Chlorpropyl)-1,2,3,4-tetrahydro-6-methyl-β-carbolin-hydrochlorid, Fp = 204 bis 205°C, Ausbeute 36,8%.

1-(γ-Chlorpropyl)-1,2,3,4-tetrahydro-6-chlor-β-carbolin-hydrochlorid, Fp = 235 bis 237°C, Ausbeute 40,6%.

Beispiel 2

1-(δ-Chlorbutyl)-1,2,3,4-tetrahydro-β-carbolin

435 Teile einer 13,7 gew.%igen δ-Chlorvaleraldehyd-Lösung in Xylol — hergestellt durch katalytisch Hydrierung einer δ-Chlorvalerylchlorid-Lösung in Xylol — wurden mit 96 Teilen 3-(β-Aminoethyl)-indol-

hydrochlorid in 500 Teilen Isopropanol 6 h bei 85°C gerührt. Nach dem Abkühlen wurden durch Absaugen 92 Teile (62,8%) 1-(δ-Chlorbutyl)-1,2,3,4-tetrahydro-β-carbolin-hydrochlorid isoliert. Nach dem Umkristallisieren hatte die Substanz einen Schmelzpunkt von 245 bis 247°C.

### Beispiel 3

1-(γ-Chlorpropyl)-1,2,3,4-tetrahydro-6-chlor-β-carbolin

86 Teile einer 17,5 gew.%igen γ-Chlorbutyraldehyd-Lösung in Xylol, 33 Teile 3-(β-Aminoethyl)-5-chlor-indol-hydrochlorid und 100 Teile Isopropanol wurden 4 h bei 82°C gerührt. Man erhielt nach dem Abkühlen, Absaugen und Waschen mit 100 Teilen Isopropanol, 27 Teile (59,4%) 1-(γ-Chlorpropyl)-1,2,3,4-tetrahydro-6-chlor-β-carbolin-hydrochlorid, Fp = 234 bis 236°C.

Die Substanz war identisch mit der analog Beispiel 1 hergestellten Verbindung.

### Beispiel 4

1-(γ-Chlorpropyl)-1,2,3,4-tetrahydro-β-carbolin

200 Teile einer 17,5 gew.%igen γ-Chlorbutyraldehyd-Lösung in Xylol, 212 Teile Isopropanol, 52 Teile 3-(β-Aminoethyl)-indol und 388 Teile einer 10 gew.%igen wäßrigen Schwefelsäure wurden 5 h bei 82°C gerührt. Nach dem Abkühlen, Absaugen und Waschen mit 100 Teilen Isopropanol erhielt man 36 Teile (31,6%) 1-(γ-Chlorpropyl)-1,2,3,4-tetrahydro-β-carbolin-hydrochlorid, Fp = 216 bis 220°C (vgl. Beispiel 1).

### Beispiel 5

1-(γ-Chlorpropyl)-1,2,3,4-tetrahydro-β-carbolin

7 Teile γ-Chlorbutyraldehyd-diethylacetal, 50 Teile Isopropanol und 7,6 Teile 3-(β-Aminoethyl)-indol-hydrochlorid wurden 7 h bei 83°C gerührt. Nach dem Abkühlen und Absaugen erhielt man 5,5 Teile (49,5%) 1-(γ-Chlorpropyl)-1,2,3,4-tetrahydro-β-carbolin-hydrochlorid, Fp = 219 bis 221°C. Die Substanz war identisch mit dem Produkt von Beispiel 1.

## Patentansprüche

1. Verfahren zur Herstellung von Salzen von 1,2,3,4-Tetrahydro-β-carbolinen der Formel I

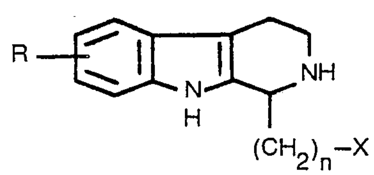

(I),

worin

R ein Wasserstoff- oder Halogenatom oder eine $C_1$—$C_4$-Alkyl-, $C_1$—$C_4$-Alkoxy- oder Trifluormethylgruppe,

X ein Halogenatom und

n eine ganze Zahl von 2 bis 5

bedeuten, dadurch gekennzeichnet, daß man ein Tryptamin der Formel II

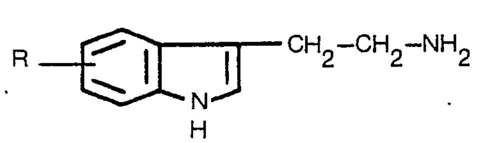

(II)

worin R die angegebene Bedeutung besitzt, oder ein Salz davon mit einem Halogenaldehyd der Formel III

$$X—(CH_2)_n—CHO \quad (III),$$

oder einem Acetal dieser Verbindung, worin X und n die angegebene Bedeutung besitzen, nötigenfalls unter Zusatz einer Säure umsetzt.

2. Verfahren gemäß Anspruch 1 zur Herstellung von Salzen der Verbindungen der Formel I gemäß Anspruch 1, in denen n die Zahl 3 bedeutet, dadurch gekennzeichnet, daß man ein Phenylhydrazin der Formel V

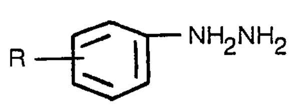

(V)

mit dem Halogenaldehyd der Formel III (n = 3) gemäß Anspruch 1 umsetzt.

**Revendications**

1. Procédé de préparation de sels de 1,2,3,4-tétrahydro-β-carbolines de formule I

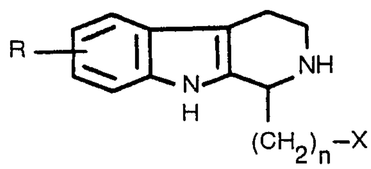

(I),

dans laquelle

R représente un atome d'hydrogène ou d'halogène ou un groupe alkyle en $C_1$—$C_4$, alcoxy en $C_1$—$C_4$ ou trifluorométhyle,

X un atome d'halogène et

n un nombre entier de 2 à 5

caractérisé par le fait que l'on fait réagir, si besoin est avec addition d'un acide, une tryptamine de formule II

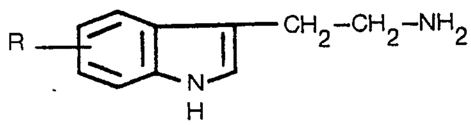

(II)

dans laquelle R a la signification indiquée, ou un sel de celle-ci, avec un aldéhyde halogéné de formule III

$$X—(CH_2)_n—CHO \qquad (III),$$

ou un acétal de ce composé, où X et n ont les significations indiquées.

2. Procédé selon la revendication 1 pour la préparation de sels des composés de formule I selon la revendication 1, dans laquelle n représente le chiffre 3, caractérisé par le fait que l'on fait réagir une phénylhydrazine de formule V

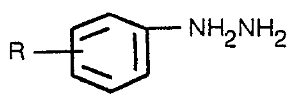

(V)

avec l'aldéhyde halogéné de formule III (n = 3) selon la revendication 1.

**Claims**

1. A process for the preparation of a salt of a 1,2,3,4-tetrahydro-β-carboline of the formula I

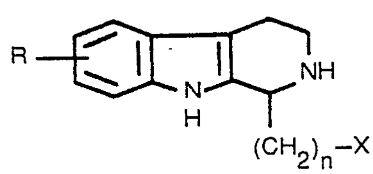

(I),

where

R is hydrogen, halogen, $C_1$—$C_4$-alkyl, $C_1$—$C_4$-alkoxy or trifluoromethyl,

X is halogen, and

n is an integer from 2 to 5, wherein a tryptamine of the formula II

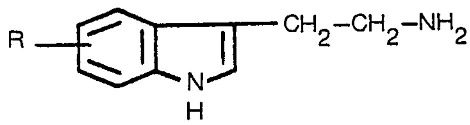

(II)

where R has the above meanings, or a salt thereof is reacted with a haloaldehyde of the formula III

$$X—(CH_2)_n—CHO \qquad (III),$$

where X and n have the above meanings, or with an acetal of this compound, if necessary with the addition of an acid.

2. A process as claimed in claim 1 for the preparation of a salt of a compound of the formula I as claimed in claim 1, in which n is 3, wherein a phenylhydrazine of the formula V

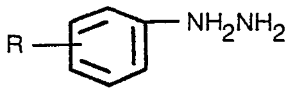

(V)

is reacted with a haloaldehyde of the formula III (n = 3) as claimed in claim 1.